# EUROPEAN PATENT APPLICATION

(11) **EP 4 640 051 A1**
(43) Date of publication of application: **29.10.2025**
(21) Application number: 24382450.5
(22) Date of filing: 24.04.2024
(51) Int. Cl.: A01N 1/02

(54) **CANISTER ASSEMBLY FOR A CRYOPRESERVATION CONTAINER**

(71) Applicant: Crioduplex, S.L., 43003 Tarragona (ES)
(72) Inventor: SAUMELL PUIG, INMACULADA, 43003 TARRAGONA (ES)
(74) Representative: Durán-Corretjer, S.L.P.

(57) **Abstract**

The present invention refers to a canister assembly (1a; 1b) for a cryopreservation container comprising: a rigid elongated handle (2) having a hook in the upper part suitable for supporting said handle in said cryopreservation container, a thermal bridge in the upper part of said handle, and a lower portion; at least two cylindrical cups (7a, 8a; 7b, 8b) having a series of apertures in the bottom side, in which said at least two cups are located one on top of the other sharing the same longitudinal axis, and being attached to said lower part of said handle; being separated by a supporting member attached in a fixed manner to the upper cylindrical cup (7a; 7b); wherein the bottom cylindrical cup (8a; 8b) is attached in a fixed manner to the lower part of the handle by fixing means (11) and the upper cylindrical cup comprises cylindrical joint means suitable for rotating the upper cylindrical cup around the handle's axis.

## Description

### Field of the invention

The present invention relates to a canister to be stored in a container for cryopreservation of biological samples, i.e. for freezing and preserving samples into a liquid nitrogen atmosphere, and which can be used in a safely and reliably manner.

### Backaround

Cryostorage tanks or containers also known as a liquid nitrogen tanks, are cylindrical stainless-steel or aluminum containers with multilayered insulated walls. The vacuum between the insulated layers prevents boiling and minimizes loss of liquid nitrogen (LN₂), allowing the tank to sustain LN₂ at a nearly steady rate. LN₂ is inert, odorless, colourless, non-corrosive, non-flammable, and extremely cold. It has been the substance of choice for cryostorage in most applications. When submerged in LN₂, cryopreserved samples are securely maintained at a temperature of -196 °C to -210 °C. The three main categories of cryostorage tanks are liquid nitrogen sample storage tanks, storage Dewar tanks (named after James Dewar) and transport storage tanks.

The process of cryopreservation is widely used for freezing and preserving samples used for reproductive purposes, but also in clinical diagnostics, immunotherapy development, food and beverage, and semiconductor storage. A wide variety of biological samples are kept in these cryostorage tanks, such as stem cells, special cells for transfusion like platelets, blood histological samples and animal biological material, as well as reproductive samples, such as sperm, embryos and oocytes.

in recent years it has been common to use *in vitro* fertilization (IVF) techniques that allow fertility treatment wherein eggs are combined with sperm outside of the human body in a laboratory. IVF involves many complex steps and is an effective form of assisted reproductive technology (ART).

The reproductive samples, such as sperm, embryos and eggs are maintained in a canister, and a plurality of canisters are stored in a cryostorage tank, which is filled up with liquid nitrogen and preserved under the environment of about -196 °C. Various types of canister assemblies have been proposed and are available in the market. One of the most common canister assemblies comprises an elongated rigid handle which hangs over the top or access passage of the cryostorage tank. The canister has a screened bottom to allow liquid refrigerant to enter into it when the canister is submerged in the refrigerant contained in the tank while preventing reproductive samples from falling out of it. Thus, the reproductive samples contained in the canister are cooled by direct contact with the refrigerant. When it is desired to remove a reproductive sample from such a canister, the canister is lifted out of the tank through the upper access opening. As this action occurs, the refrigerant flows out of the canister's screened bottom. Thus, in order to remove a single reproductive sample from such a canister assembly, it is necessary to take all of the reproductive samples in the canister out of contact with the refrigerant. Herein lays the major disadvantage of such canister assemblies. That is, certain types of reproductive samples warm up and thaw quickly. Hence, even a short "out-of-refrigerant contact time" can be harmful to sperm, embryos and eggs contained in such samples.

The canisters are made of any material resistant to LN₂, for example stainless steel, also known as inox. Stainless steel holds up better to the extreme cold conditions inside the cryostorage tank.

At the bottom of the cryostorage container there is the spider. It is a small piece with slots that allow the bottom of the canisters to rest against it. This keeps the canisters from rattling when the tank is being moved and helps to keep them in place when removing and replacing them.

in view of the above, there is still the need to provide a canister assembly for a cryopreservation container capable of accommodating a higher amount of in the same cryopreservation container or tank. Therefore, it is an object of this invention to provide a canister assembly that keeps all the advantages of the canisters already known but allowing to increase the amount of samples that can be stored in the cryopreservation container, to minimize loss of liquid nitrogen (LN₂) and to reduce the number of cryopreservation containers to be used. All this promotes a more efficient and sustainable environmental impact and contributes to a more sustainable future.

### Description of the invention

The words "comprises/comprising" and the words "having/including" when used herein with reference to the present invention are used to specify the presence of stated features, integers, steps or components but do not preclude the presence or addition of one or more other features, integers, steps, components or groups thereof.

It should be appreciated by those skilled in the art that the specific embodiments disclosed herein should not be read in isolation, and that the present specification intends for the disclosed embodiments to be read in combination with one another as opposed to individually. As such, each embodiment may serve as a basis for modifying or limiting other embodiments disclosed herein.

As used in this specification and claims, the singular forms "a," "an" and "the" include plural references unless the content clearly dictates otherwise.

in a first aspect, the present invention refers to a canister assembly for a cryopreservation container (hereinafter referred to as "storage container") for preparing, storing and transporting a frozen biological sample, in particular sperm, eggs or embryos in the field of assisted reproductive technology (ART).

The canister assembly of the present invention comprises at least two cylindrical cups sharing the same longitudinal axis, being the bottom cup attached in a fixed manner to an elongate handle, and having the other cups eccentric rotatory movement allowing access to the immediately inferior cup. The canister assembly of the present invention allows increasing the storage capacity of a cryopreservation container for a comfortable manipulation by the user and easy location of samples. In addition, said canister assembly can be used in almost all known cryopreservation tanks in the market, reducing the amount of the cryopreservation liquid to be used or reducing the number of cryopreservation tanks to be used for a number of biological samples.

Different ways to monitor the contents of the cryopreservation liquid in a cryopreservation tank have been developed. Nowadays, research facilities or clinics have effective local alarms and monitoring systems that can continuously monitor the temperature or the level of liquid nitrogen in the cryopreservation tanks. Upon detection of a deviation past the level or temperature set limits, the system is able to auto-dial and alert staff during and out of working hours. Therefore, the cryopreservation environment is kept over time, and therefore samples can be used in a safely and reliably manner.

Another important parameter in the field of cryopreservation is the liquid nitrogen evaporation rates of the container. The canister assembly of the present invention can be adapted to any type of container available on the market since the upper portion of the canister handle is not modified in the present invention. This makes it possible to guarantee the evaporation rate stipulated by the container manufacturer.

Thus, the present invention relates to a canister assembly capable of organizing and storing a higher amount of cryopreserved samples which at the same time allows taking out the cryopreserved samples in a safe and quick manner. The canister assembly of the present invention suitable for a cryopreservation container comprising: a rigid elongated handle having a hook in the upper part suitable for supporting said rigid elongated handle in an a cryopreservation container, a thermal bridge in the upper part of said elongated handle and a lower portion; at least two cylindrical cups having a series of apertures in the bottom side, in which said at least two cylindrical cups are located one on top of the other sharing the same longitudinal axis, and being attached to said lower part of said rigid elongated handle; being separated by a supporting member attached in a fixed manner to the upper cylindrical cup; characterized in that the bottom cylindrical cup is attached in a fixed manner to the lower part of the handle by fixing means and the upper cylindrical cup comprises cylindrical joint means suitable for rotating the upper cylindrical cup around the handle's axis.

As used herein, the term "canister" means a round or cylindrical container used for storing biological samples such as stems cells, special cells for transfusion like platelets, blood, histological samples and animal biological material, as well as reproductive samples, such as sperm, embryos and fertilized eggs.

As used herein, the term "cylindrical cup" means a vessel or recipient in the shape of a cylinder, with a circular cross section. However, the cups of the present invention can be of any cross section shape including elliptical, rectangular, square, hexagonal, triangular, or any other geometric shape suitable for containing samples.

The rigid elongated handle has two portions: the upper portion comprising a hook and a thermal bridge and the lower portion having attached the at least two cylindrical cups. The handle may be sufficiently rigid to enable the canister to be handled by the upper portion, for instance enabling a user to remove the canister from a container by grasping the hook of the upper portion of the handle. The joint between the upper portion and the lower portion of the handle can be a bend such as a "Y shape" or can be straight, depending on the type of the container and the size of the container inlet orifice where the canister is stored.

As used herein, the term "thermal bridge", also called a cold bridge, heat bridge, or thermal bypass, is an area or component of an object which has higher thermal conductivity than the surrounding materials, creating a path of least resistance for heat transfer. Thermal bridges result in an overall reduction in thermal resistance of the object and improving comfort of the investigator or working staff.

The cylindrical cups have a series of apertures or drain holes in form of a screened bottom to allow liquid refrigerant to enter into it when the canister is submerged in the refrigerant contained in the container while preventing biological samples from falling out of it, and flows out of the canister's screened bottom when the canister is lifted out of the tank through the upper access opening.

As used herein, the term "cylindrical joint means" means that is a joint that allows that the handle is not fixed to the cylindrical cup in order to obtain a cylindrical cup able to rotate around the handle.

Preferably, the at least one upper cylindrical cup has two cylindrical joint means.

Preferably, the upper part of the rigid elongated handle is attached to the lower part of the rigid elongated handle in a form that the handle is adapted to the cryopreservation container. More preferably, the upper part of the rigid elongated handle is attached to the lower part of the rigid elongated handle in a "Y" form. More preferably, the upper part of the rigid elongated handle is attached to the lower part of the rigid elongated handle in a straight form.

Preferably, the canister assembly has at least two cylindrical cups. More preferably, the canister has two, three, four, five, six, seven, eight, nine, ten cylindrical cups. Even more preferably, the canister has three cylindrical cups. The most preferably, the canister has two cylindrical cups.

Preferably, said supporting member comprises a translation blocker surface.

As used herein, the term "translation blocker surface" means that is a piece that allows the movement of the upper cylindrical cup in a straight line along a single axis side to side avoiding that said cylindrical cup fall towards the bottom cylindrical cup. In particular, the translation blocker surface of this invention is a piece of metal, preferably of stainless steel.

Preferably, said supporting member comprises a rotation locker surface.

As used herein, the term "rotation locker surface" means that is a piece that blocks the rotation of the upper cylindrical cup when in a lock position while the canister is inside the container or when no samples need to be grabbed. In particular, the rotation locker surface of this invention is a piece of metal, preferably of stainless steel.

Preferably, the translation blocker and the rotation locker are the same piece of metal, preferably of stainless steel.

As used herein, the term "fixing means" means that is means of attaching the bottom cylindrical cup to the handle. By using said means, all translation or rotation movements are suppressed.

Preferably, said bottom cylindrical cup has at least one cylindrical joint mean.

Preferably, said bottom cylindrical cup has one cylindrical joint mean and one fixing mean.

Preferably, the canister assembly is made of any material resistant to LN₂. More preferably, the canister assembly is made of stainless steel.

On the other hand, the present invention refers to the use of the canister assembly for being stored in a container for cryopreservation.

Preferably, the present invention refers to the use of the canister assembly in which said biological sample is selected from the list comprising stem cells, special cells for transfusion like platelets, blood, histological samples and animal biological material, as well as reproductive samples, such as sperm, embryos and fertilized eggs.

A series of drawings representing at least one embodiment of the canister assembly object of the present invention are appended to ensure better understanding through explanatory but not limiting examples.
Fig. 1 is a 3D view of one embodiment of a canister assembly according to the present invention.
Fig. 2 is a 3D view of two different embodiments of the canister assembly according to the present invention.
Fig. 3 is a rear 3D view of a canister assembly embodiment according to the present invention.
Fig. 4 is a top 3D view of a canister assembly embodiment according to the present invention.
Fig. 5 is detailed 3D view of the supporting member in the canister assembly according to the present invention.
Fig. 6 is a lateral 3D view of a canister assembly having three cylindrical cups according to the present invention.
Fig. 7 is a cross-sectional view of the canister assembly having a "Y" shape handle inside a container for a cryopreservation.
Fig. 8 is a cross-sectional view of the canister assembly having three cylindrical cups and having a straight handle inside a container for a liquid gas refrigerant cryopreservation.

in the figures, the same or equivalent elements have been identified with identical numerals.

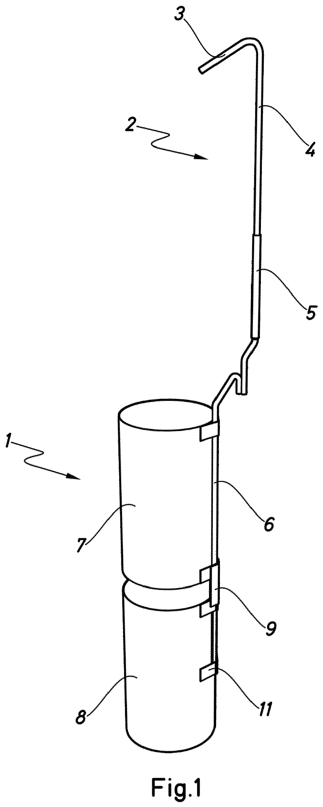

As can be seen from Fig. 1, the canister assembly (1) includes a handle (2), which has an upper portion (4) including a hook (3) and a thermal bridge (5). The handle (2) allows manipulation of the canister assembly (1) inside or outside a cryopreservation container. The hook (3) at the upper part (4) of the handle (2) allows the canister assembly (1) to rest on the neck of a cryopreservation container. The handle (2) also comprises a lower portion (6) to support at least two cylindrical cups (7, 8). In the particular embodiment shown in Fig. 1, the handle (2) is provided as a bend in the joint of the upper portion (4) and lower portion (6) of the handle (2) having a "Y" shape. The canister assembly (1) comprises two cylindrical cups (7, 8) wherein the upper cylindrical cup (7) is attached to the lower portion (6) of said handle (2) in a cylindrical manner by cylindrical joint means (10) and the bottom cylindrical cup (8) is attached to said handle (2) in a fixed manner by fixing means (11). Between the upper cylindrical cup (7) and the bottom cylindrical cup (8) there is a supporting member (9).

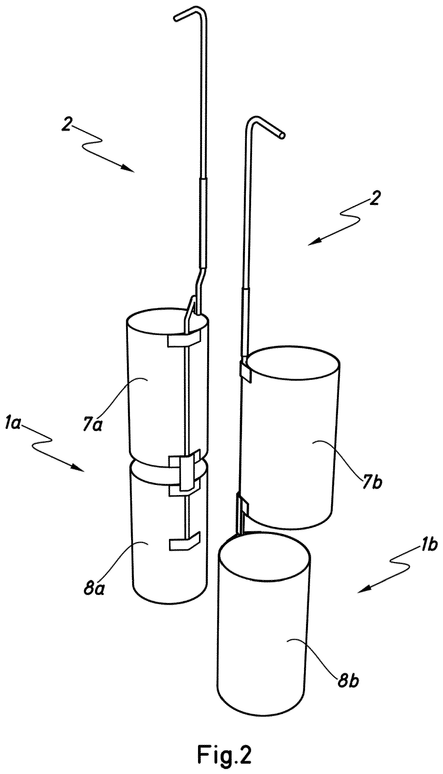

Fig. 2 shows two canisters assemblies (1a, 1b). The canister assembly (1a) having a "Y" shape handle (2) is represented in a locked position, which is the position that canister assembly (1a) has, for example, when it is inside a container. In this position, both cylindrical cups (7a, 8a) are arranged vertically on top of each other sharing the same longitudinal axis. The canister assembly (1b) has a straight handle (2) and the bottom cylindrical cup (8b) is fixed to the handle (2) whereas the upper cylindrical cup (7b) is unlocked and therefore it is eccentrically rotated around the handle's (2) axis. This position is used when it is desired to remove a biological sample from such a canister (1b), the canister assembly (1b) is lifted out of the container through the access opening. As this action occurs, the user needs to remove the canister assembly (1b) from the container and raise the upper cylindrical cup (7b) upwards in order to unlock the upper cylindrical cup (7b) from its locked position. This unlocking allows the upper cylindrical cup (7b) to rotate eccentrically around the handle's (2) axis in order to extract a biological sample from the bottom cylindrical cup (8b). After the biological sample is taken, the upper cylindrical cup (7b) can be returned to the locked position raising it upward and positioning the upper cylindrical cup (7b) on top of the bottom cylindrical cup (8b), as shown for cylindrical cups 7a and 8a.

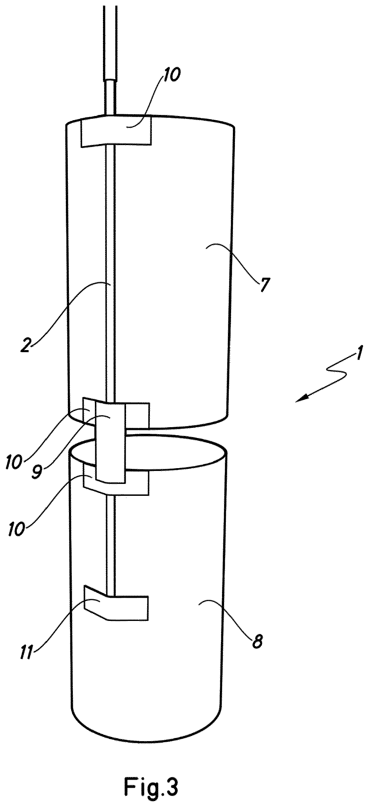

Fig. 3 shows the canister assembly (1) at the rear. Fig. 3 shows that the handle (2) runs along the rear of the cylindrical cups (7, 8). Between the upper cylindrical cup (7) and the bottom cylindrical cup (8) there is a supporting member (9) that connects both cylindrical cups (7, 8) and acts as a stop so that the upper cylindrical cup (7) does not touch the bottom cylindrical cup (8), delimiting a space between both cylindrical cups (7, 8). The upper cylindrical cup (7) has two cylindrical joint means (10) one at the top and one at the bottom of the cylindrical cup (7). The bottom cylindrical cup (8) has one cylindrical joint mean (10) at the top of the cylindrical cup (8) and one fixing mean (11) in the lower portion (6) of the handle (2).

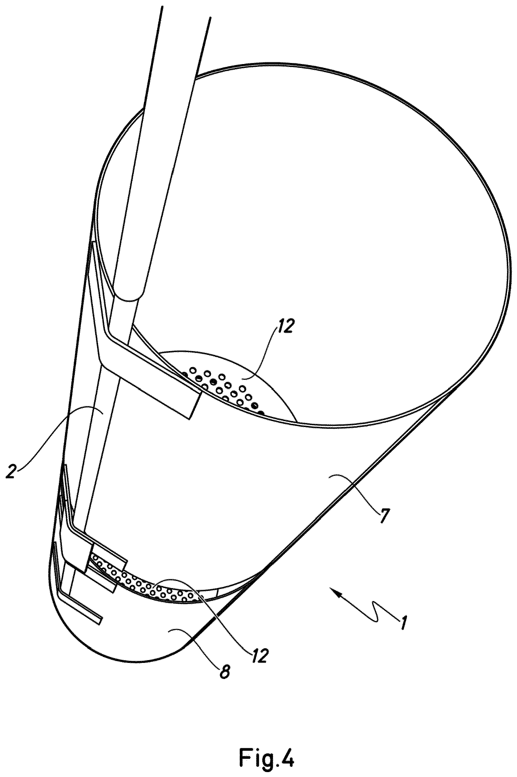

Fig. 4 depicts a representative embodiment of the canister assembly (1) of the present invention in a cross-sectional view. The cylindrical cups (7, 8) have a series of apertures or drain holes (12) in form of a screened bottom to allow the cryogenic liquid, for example, liquid nitrogen to drain when the canister assembly (1) is taken out from the cryopreservation container. Also Fig. 4 shows that the handle (2) is attached but not fixed at the upper cylindrical cup (7), allowing the upper cylindrical cup (7) to rotate eccentrically around the handle's (2) axis.

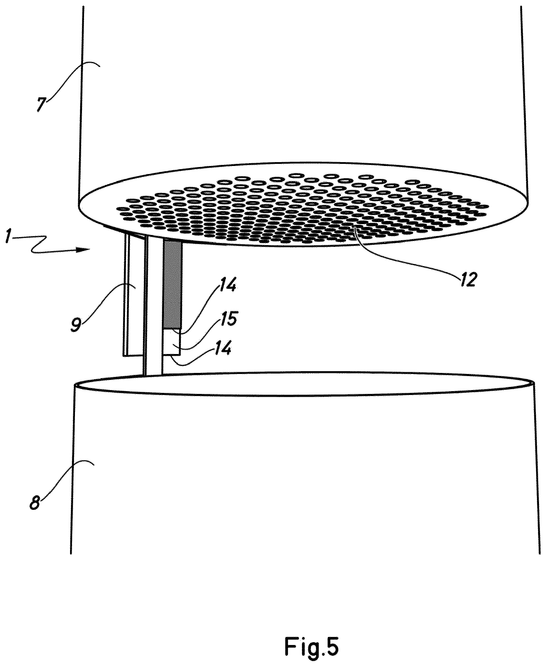

Fig. 5 shows that the supporting member (9) is a piece that consists in two surfaces. The first surface is a translation blocker surface (14) for locking the movement of the upper cylindrical cup in a straight line along a single axis side to side avoiding that said cylindrical cup to fall towards the bottom cylindrical cup. The second surface is a rotation locker surface (15) for locking rotation along the handle's axis when the upper cylindrical cup (7) is positioned on top of the bottom cylindrical cup (8), for example, not allowing the canister assembly (1) to rotate when it is inside the container or when no samples need to be grabbed.

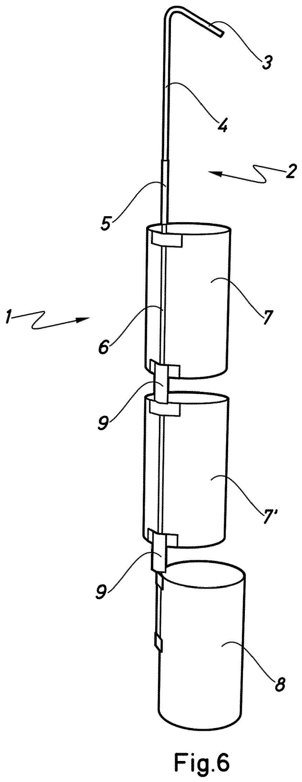

Fig. 6 shows an embodiment of canister assembly (1) according to the present invention. The canister assembly (1) has a bottom cylindrical cup (8) in a fixed position whereas the upper cylindrical cups (7, 7') are unlocked and therefore eccentrically rotated around the handle's (2) axis. The canister assembly (1) comprises three cylindrical cups (7, 7', 8). The canister assembly (1) has two upper cylindrical cups (7, 7') that are able to rotate and one bottom cylindrical cup (8) which is fixed and it is situated in the lower part of the canister assembly (1). There is a supporting member (9) between the upper cylindrical cup (7) and the middle cylindrical cup (7') and between the middle cylindrical cup (7') and the bottom cylindrical cup (8).

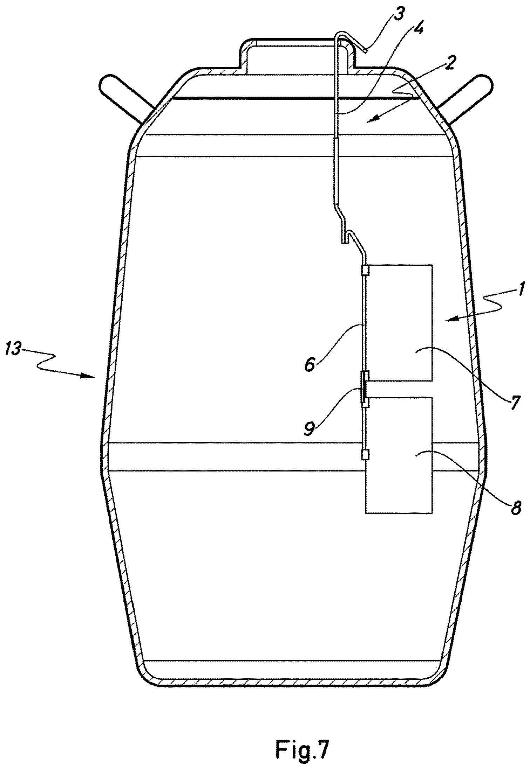

Fig. 7 shows an embodiment of a canister assembly (1) according to the present invention having two cylindrical cups (7, 8) inside of a portable Dewar container (13). The canister assembly (1) includes a handle (2), which has a "Y" form. The handle (2) allows manipulation inside a container and has a hook (3) at the upper part (4) that allows the canister assembly (1) to rest on the neck of the Dewar container (13). The canister assembly (1) comprises two cylindrical cups (7, 8) wherein the upper cylindrical cup (7) is attached by cylindrical join means and the bottom cylindrical cup (8) is attached by fixing means to said rigid elongated handle (2). Between the upper cylindrical cup (7) and the bottom cylindrical cup (8) there is a supporting member (9).

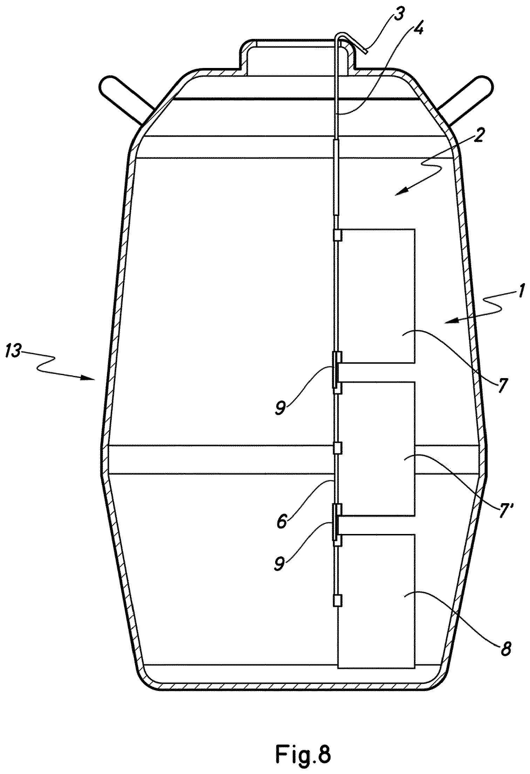

Fig. 8 shows a canister assembly (1) having three cylindrical cups (7, 7', 8) inside of a Dewar container (13). The canister assembly (1) includes a handle (2), which has a straight form. The handle (2) allows manipulation inside a container and has a hook (3) at the upper part (4) that allows the canister assembly (1) to rest on the neck of the Dewar container (13). The canister assembly (1) comprises three cylindrical cups (7,7',8) wherein the upper cylindrical cups (7,7') are attached by cylindrical join means and the bottom cylindrical cup (8) is attached by fixing means to said rigid elongated handle (2). There is a supporting member (9) between the upper cylindrical cup (7) and the middle cylindrical cup (7') and between the middle cylindrical cup (7') and the bottom cylindrical cup (8).

Although this disclosure is in the context of certain embodiments and examples, those skilled in the art will understand that the present disclosure extends beyond the specifically disclosed embodiments to other alternative embodiments and/or uses of the embodiments and obvious modifications and equivalents thereof. In addition, while several variations of the embodiments have been shown and described in detail, other modifications, which are within the scope of this disclosure, will be readily apparent to those of skill in the art based upon this disclosure.

It is also contemplated that various combinations or sub-combinations of the specific features and aspects of the embodiments may be made and still fall within the scope of the disclosure. It should be understood that various features and aspects of the disclosed embodiments can be combined with, or substituted for, one another in order to form varying modes or embodiments of the disclosure. Thus, it is intended that the scope of the present disclosure herein disclosed should not be limited by the particular disclosed embodiments described above.

It should be understood, however, that this description, while indicating preferred embodiments of the disclosure, is given by way of illustration only, since various changes and modifications within the spirit and scope of the disclosure will become apparent to those skilled in the art.

It should be appreciated by those skilled in the art that the specific embodiments disclosed within paragraphs [0036] - [0053] should not be read in isolation, and that the present specification intends for these embodiments to be disclosed in combination with other embodiments as opposed to being disclosed individually. For example, each of the embodiments disclosed in paragraphs [0015] - [0035] is to be read as being explicitly combined with each of the embodiments in paragraphs [0036] - [0053], or any permutation of 2 or more of the embodiments disclosed therein.

The terminology used in the description presented herein is not intended to be interpreted in any limited or restrictive manner. Rather, the terminology is simply being utilized in conjunction with a detailed description of embodiments of the systems, methods and related components. Furthermore, embodiments may comprise several novel features, no single one of which is solely responsible for its desirable attributes or is believed to be essential to practicing the embodiments herein described.

Although the canister assembly shown above is configured for a liquid nitrogen refrigerant cryopreservation container for freezing and preserving specimens of reproductive samples such as fertilized eggs, vitrified embryos and sperm, said canister can also be used for clinical diagnostics, immunotherapy development, food and beverage, and semiconductor storage. The canister is specially configured to work, among others, with stem cells, special cells for transfusion like platelets, blood, histological samples and animal biological material and other types of products for medical and/or pharmaceutical use.

Although the invention was presented and described in reference to its embodiments, it is understood that these have no limiting effect on the invention, so that multiple structural details or others that may be obvious for a person skilled in the art may vary after interpreting the subject matter that is disclosed in the present description, claims and drawings. In particular, in principle and unless explicitly stated otherwise, all the features of each of the different embodiments and alternatives shown and/or suggested can be combined with one another. Therefore, all the variants and equivalents will fall within the scope of the present invention if they can be considered to be comprised in the broader scope of the following claims.

## Claims

1. A canister assembly (1) suitable for a cryopreservation container comprising:
- a rigid elongated handle (2) having a hook (3) in the upper part (4) suitable for supporting said rigid elongated handle (2) in an a cryopreservation container (13), a thermal bridge (5) in the upper part (4) of said elongated handle (2) and a lower portion (6);
- at least two cylindrical cups (7,8) having a series of apertures (12) in the bottom side, in which said at least two cylindrical cups (7,8) are located one on top of the other sharing the same longitudinal axis, and being attached to said lower part (6) of said rigid elongated handle (2); being separated by a supporting member (9) attached in a fixed manner to the upper cylindrical cup (7);
**characterized in that** the bottom cylindrical cup (8) is attached in a fixed manner to the lower part (6) of the handle (2) by fixing means (11) and the upper cylindrical cup (7) comprises cylindrical joint means (10) suitable for rotating the upper cylindrical cup (7) around the handle's (2) axis.

2. Canister assembly, according to claim 1, **characterized in that** the upper part (4) of the rigid elongated handle (2) is attached to the lower part (6) of the rigid elongated handle (2) in a "Y" form.

3. Canister assembly, according to claim 1 or 2, **characterized in that** the upper part (4) of the rigid elongated handle (2) is attached to the lower part (6) of the rigid elongated handle (2) in a straight form.

4. Canister assembly, according to any one of the preceding claims, **characterized in that** said canister assembly has two or three cylindrical cups.

5. Canister assembly, according to claim 4, **characterized in that** said canister assembly has two cylindrical cups.

6. Canister assembly, according to any of the preceding claims, **characterized in that** said supporting member (9) comprises a translation blocker surface (14).

7. Canister assembly, according to any of the preceding claims, **characterized in that** said supporting member (9) comprises a rotation locker surface (15).

8. Canister assembly, according to any of the preceding claims, **characterized in that** said bottom cylindrical cup (8) has at least one cylindrical joint mean (10).

9. Canister assembly, according to any of the preceding claims, **characterized in that** the canister assembly is made of any material resistant to LN₂.

10. Canister assembly, according to any of the preceding claims, **characterized in that** the canister assembly is made of stainless steel.

11. Use of the canister assembly according to any of the preceding claims for storing of biological samples in a container for cryopreservation.

12. Use, according to claim 11, **characterized in that** said biological sample is selected from the list comprising stem cells, special cells for transfusion like platelets, blood, histological samples and animal biological material, as well as reproductive samples, such as sperm, embryos and fertilized eggs.
